# EUROPEAN PATENT SPECIFICATION

(11) **EP 2 140 270 B1**
(45) Date of publication and mention of the grant of the patent: **17.12.2014**
(21) Application number: 08719010.4
(22) Date of filing: 09.04.2008
(51) Int. Cl.: G01N 33/68

(54) **METHOD OF MASS SPECTROMETRY**
VERFAHREN ZUR MASSENSPEKTROMETRIE
PROCÉDÉ DE SPECTROMÉTRIE DE MASSE

(30) Priority: 13.04.2007 GB 0707165
(43) Date of publication of application: 06.01.2010
(73) Proprietor: Micromass UK Limited, Stamford Avenue Altrincham Road Wilmslow SK9 4AX (GB)
(72) Inventor: GOODLETT, David, R., Seattle, WA 98199 (US); HUGHES, Chris, Manchester M29 7BJ (GB); LANGRIDGE, James, Ian, Sale, Cheshire M33 5BZ (GB)
(74) Representative: Jeffrey, Philip Michael
(86) International application number: PCT/GB2008/001270
(87) International publication number: WO 2008/125821

(56) References cited:
- MCLEAN J A ET AL: "Ion mobility-mass spectrometry: a new paradigm for proteomics" INT J MASS SPECTROM, vol. 240, no. 3, 1 February 2005 (2005-02-01), pages 301-315, XP004711566
- RUOTOLO B T ET AL: "Analysis of phosphorylated peptides by ion mobility-mass spectrometry." ANAL CHEM, vol. 76, no. 22, 15 November 2004 (2004-11-15), pages 6727-6733, XP002486732
- TRAKSELIS M A ET AL: "Identification and mapping of protein-protein interactions by a combination of cross-linking, cleavage, and proteomics." BIOCONJUG CHEM, vol. 16, no. 4, July 2005 (2005-07), pages 741-750, XP002486733
- SINZ A: "Chemical cross-linking and mass spectrometry to map three-dimensional protein structures and protein-protein interactions." MASS SPECTROM REV, vol. 25, no. 4, July 2006 (2006-07), pages 663-682, XP002486734
- COLLINS D C ET AL: "Developments in ion mobility spectrometry-mass spectrometry." ANAL BIOANAL CHEM, vol. 372, no. 1, January 2002 (2002-01), pages 66-73, XP002486735

## Description

The present invention relates to a method of mass spectrometry. The preferred embodiment relates to a method of determining protein interaction partners and optionally the site of interaction. According to the preferred embodiment ions are subjected to ion mobility separation followed by mass spectrometry in order to identify cross-linked peptides.

The biological function of proteins is through complex interactions with other proteins, DNA and RNA and it is believed that these protein-protein or protein-biomolecular interactions may well play a pivotal role in important areas of biology. For example, disease may result from perturbations of these biological interactions and by studying these interactions it may well prove possible to garner a significantly improved understanding of biological processes and the nature and progression of diseases.

Protein-protein interactions are recognised as the molecular mechanism by which complex biological processes are often affected. Interaction between intracellular proteins can be both subtle and short-lived with two or more constituent proteins associating in the catalysis of a step in a biosynthetic or signal-transduction pathway or the formation of stable multiprotein complexes which perform known biological functions.

A known method of determining protein interactions is by chemical cross-linking. The basis of this technique is the formation of covalent bonds between two proteins using bifunctional reagents containing reactive end groups. The reactive end groups react with peptide or protein functional groups such as primary amines, carboxyl group and sulfhydryls. This technique has primarily been used *in vitro.* If two proteins interact physically with each other then they can be covalently cross-linked. The formation of cross-links between two distinct proteins is a direct and convincing evidence of their close proximity. In addition to information on the identity of the interacting proteins, cross-linking experiments can also reveal the regions or amino acid sites of contact between them.

Cross-linking agents or cross-linkers may comprise either homo- or heterobifunctional reagents with identical or non-identical reactive groups permitting the establishment of inter-molecular as well as intra-molecular cross-linkages.

Protein-protein interactions have been studied using methods such as X-ray crystallography and Nuclear Magnetic Resonance (NMR) spectroscopy. These approaches have provided insight into the spatial and topological organization of proteins and protein complexes. However, NMR spectroscopy is problematic in that this approach requires relatively large quantities of purified protein in specific solvents. X-ray investigation is also problematic in that it requires that the protein should be crystallisable.

At present, the best definition of the atomic interfaces in protein-protein complexes depends upon the use of physical techniques such as chemical cross-linking and mass spectrometry. Chemical cross-linking helps to stabilise covalently the interactions of protein-protein complexes. It is known, for example, to chemically cross-link two proteins and to subject the resulting protein-protein complex to gel electrophoresis (in order to separate the cross-linked species) and then to enzymatic digestion in order to yield a mixture of cross-linked and non cross-linked peptides.

It is also known to perform proteolysis in H2O16 and H2O18 followed by MS analysis in order to aid the detection and subsequent identification of cross-linked peptides. Such an approach is, for example, disclosed in Gao et al., Journal of Biological Chemistry, Vol. 281, No. 29, pp. 20404-20417, 21 July 2006.

It has been proposed to identify protein-protein interactions by a combination of cross-linking and mass spectrometry in Bioconjugate Chemistry, Vol. 16, No. 4, pp. 741-750, July 2005.

However, in practice, as with the detection and sequencing of phosphopeptides, cross-linked peptides if present in a sample are normally present at relatively low stoichiometry relative to other peptides present in a sample and are hence remain difficult to observe and recognise.

Ion mobility-mass spectrometry is a technology that is used for the identification of the presence of covalently bound small entities such as post-translational modifications, e.g. phosphorylations, or modifications with other small molecules, e.g. heme (McLean et al., International Journal of Mass Spectrometry, Vol. 240, No. 3, pp. 301-315, 2005). The study of intact protein complexes represents a further application of the technology (McLean et al., *vide supra).*

It is desired to provide an improved method of identifying and investigating protein-protein and protein-biomolecular interactions.

According to an aspect of the present invention there is provided a method of mass spectrometry comprising:
enzymatically digesting, chemically reacting or fragmenting a covalently cross-linked protein complex to form or produce a plurality of peptides, wherein the peptides comprise a mixture of cross-linked peptides and non cross-linked peptides;
ionising at least some of the plurality of peptides to form a plurality of peptide ions;
separating at least some of the peptide ions according to their ion mobility or their rate of change of ion mobility with electric field strength and obtaining ion mobility data; and
mass analysing at least some of the peptide ions and
obtaining mass spectral data.

According to an embodiment the method further comprises distinguishing between cross-linked peptides or cross-linked peptide ions and non cross-linked peptides or non cross-linked peptide ions. The method preferably further comprises: (i) determining, recognising or identifying one or more cross-linked peptides or cross-linked peptide ions; and/or (ii) determining, recognising or identifying one or more non cross-linked peptides or non cross-linked peptide ion.

According to the preferred embodiment the method further comprises the step of determining, recognising or identifying one or more cross-linked peptides or cross-linked peptide ions by analysing the ion mobility data and/or the mass spectral data. According to an embodiment the method further comprises the step of determining, recognising or identifying the one or more non cross-linked peptides or non cross-linked peptide ions by analysing the ion mobility data and/or the mass spectral data.

According to an embodiment the method further comprises analysing the ion mobility data and/or the mass spectral data to determine, recognise or identify peptides or peptide ions which have:
(i) an ion mobility characteristic and/or a mass spectral characteristic which falls outside of a standard, normal or predicted range; and/or
(ii) an ion mobility characteristic and/or a mass spectral characteristic which falls towards an upper limit or towards a lower limit of a standard, normal or predicted range; and/or
(iii) an outlying ion mobility characteristic and/or an outlying mass spectral characteristic; and/or
(iv) an ion mobility drift time versus mass to charge ratio relationship or dependence which falls between or outside one or more ranges or bands predicted for non cross-linked peptides; and/or
(v) an ion mobility drift time versus mass to charge ratio relationship or dependence which falls within one or more ranges or bands predicted for cross-linked peptides.

According to an embodiment the method further comprises analysing the ion mobility data and/or the mass spectral data:
(i) to determine, recognise or identify peptides or peptide ions which have an ion mobility drift time which is x% faster or slower (or substantially faster or slower) than the average or mean predicted drift time for non cross-linked ions or other ions having a comparable mass to charge ratio and/or charge state; and/or
(ii) to determine, recognise or identify peptides or peptide ions which have a mass or mass to charge ratio which is x% higher or lower (or substantially higher or lower) than the average or mean predicted or experimentally determined mass or mass to charge ratio for non cross-linked ions or other ions having a comparable drift time or ion mobility.

Preferably, x is selected from the group consisting of: (i) < 5; (ii) 5-10; (iii) 10-15; (iv) 15-20; (v) 20-25; (vi) 25-30; (vii) 30-35; (viii) 35-40; (ix) 40-45; (x) 45-50; (xi) 50-55; (xii) 55-60; (xiii) 60-65; (xiv) 65-70; (xv) 70-75; (xvi) 75-80; (xvii) 80-85; (xviii) 85-90; (xix) 90-95; (xx) 95-100; (xxi) 100-110; (xxii) 110-120; (xxiii) 120-130; (xxiv) 130-140; (xxv) 140-150; (xxvi) 150-160; (xxvii) 160-170; (xxviii) 170-180; (xxix) 180-190; (xxx) 190-200; and (xxxi) > 200.

According to an embodiment the method may further comprise the step of forming the cross-linked protein complex prior to the step of enzymatically digesting, chemically reacting of fragmenting the cross-linked protein complex.

According to an embodiment the step of forming the cross-linked protein complex may comprise reacting an analyte protein with a reagent to form a cross-linked protein complex. Preferably, two proteins are linked by one or more bridges being formed between the two proteins. The reagent is preferably selected from the group consisting of: (i) Disuccinimidyl tartarate; (ii) *bis*-sulfosuccinimidyl suberate ("BS3 or Sulfo-DSS"); (iii) Ethylene glycol bis(sulfosuccinimidyl) succinate; (iv) 3,3'-dithiobis(sulfosuccinimidyl propionate) ("DTSSP"); (v) dimethyl suberimidate; (vi) formaldehyde; (vii) 1-Ethyl-3-[3-dimethylaminopropyl]carbodiimide hydrochloride ("EDC or EDAC"); (viii) dimethyl dithiobispropionimidate ("DTBP"); (ix) DiSuccinimidyl suberate; (x) *N,N*'1,3-phenylenedimaleimide ("mPDM"); (xi) *N,N*'-1,2-phenylenedimaleimide ("oPDM"); (xii) *N,N*'-1,4-phenylenedimaleimide ("pPDM"); (xiii) *N,N*'-(methylene-4-1-phenylene)bismaleimide ("BM"); (xiv) naphthalene-1,5-dimaleimide ("NDM"); (xv) bismaleimidoethane ("BMOE"); (xvi) 1,4-bismaleimidobutane ("BMB"); (xvii) 1,4-bismaleimidyl-2,3-dihydroxybutane ("BMDB"); (xviii) 1,6-bismaleimidohexane ("BMH"); (xix) dithio-bis-maleimdoethane ("DTME"); (xx) 1,8-bis-maleimidotriethyleneglycol ("BM[PEO]₃"); (xxi) 1,11-bis-maleimidotetraethyleneglycol ("BM[PEO]₄"); (xxii) C-3 disulfide; (xxiii) C-4 disulfide; (xxiv) C-6 disulfide; (xxv) C-7 disulfide; (xxvi) C-8 disulfide; (xxvii) C-9 disulfide; (xxviii) 1,4-di-(3'-[2-pyridyldithio]-propionamido) butane ("DPDPB"); (xxix) dimethyl adipimidate ("DMA"); (xxx) dimethyl pimelimidiate ("DMP"); (xxxi) dimethyl suberimidate ("DMS"); (xxxii) N-benzyliminodiacetoyloxysuccinimid ("BID"); (xxxiii) disuccinimidyl glutarate ("DSG"); (xxxiv) dithiobis(succinimidylpropionate) ("DSP"); (xxxv) disuccinimidyl suberate ("DSS"); (xxxvi) disuccimimidyl tartarate ("DST"); (xxxvii) ethyleneglycol bis-(succinimidylsuccinate) ("EGS"); (xxxviii) bis(2-[succinimidooxycarbonyloxy]ethyl)sulfone ("BSOCOES"); (xxxix) 1,5-difluro-2,4-dinitrobenzene ("DFDNB"); (xl) 4,4'-difluoro3,3'-dinitrodiphenylsulfone ("DFDNPS"); (xli) dibromobimane ("bBBr"); (xlii) Succinimidyl-4-(N-maleimidomethyl)cyclohexane-1-carboxylate ("SMCC"); (xliii) (Sulfosuccinimidyl 6-(3'-[2-pyridyldithio]-propionamido)hexanoate) ("Sulfo-LC-SPDP"); and (xliv) Dithiobis(succinimidyl)propionate ("DTSP or DSP").

The step of forming the cross-linked protein complex may comprise either:
(i) heating an analyte protein sample; and/or
(ii) pressurising an analyte protein sample; and/or
(iii) radiating an analyte protein sample; and/or
(iv) exposing an analyte protein sample to an electron beam, gamma-radiation or ultraviolet light; and/or
(v) exposing an analyte protein sample to a photosensitiser.

The step of forming the cross-linked protein complex may comprise reacting an analyte protein sample with one or more proteins, nucleic acids, DNA oligomers, RNA molecules, oligionucleotides, single-strand binding proteins ("SSB or SSBP") or DNA binding proteins. The step of reacting the analyte protein sample with nucleic acid may further comprise irradiating the analyte protein sample and the nucleic acid with ultra-violet radiation to induce the cross-linked protein complex.

The cross-linked protein complex comprises at least two polymer chains linked to each other via one or more bridges formed between the two polymer chains by covalent cross-linking bonds.

The method preferably further comprises operating in a MS/MS or tandem mass spectrometry mode of operation which preferably comprises repeatedly switching between a first mode of operation and a second mode of operation, wherein:
in the first mode of operation the method comprises the steps of:
   (i) separating at least some of the plurality of peptide ions according to their ion mobility or rate of change of ion mobility with electric field strength and obtaining ion mobility data; and
   (ii) mass analysing at least some of the peptide ions and obtaining mass spectral data;
and wherein in the second mode of operation the method comprises the steps of:
   (i) fragmenting or reacting at least some or substantially all of the plurality of peptide ions in a collision, fragmentation or reaction device to form a plurality of fragment, daughter, product or adduct ions; and
   (ii) mass analysing the fragment, daughter, product or adduct ions.

The method may further comprise operating in a Shotgun fragmentation mode of operation which preferably comprises repeatedly switching between a first mode of operation and a second mode of operation, wherein:
in the first mode of operation the method comprises the steps of:
   (i) separating at least some of the plurality of peptide ions according to their ion mobility or rate of change of ion mobility with electric field strength and obtaining ion mobility data; and
   (ii) mass analysing at least some of the peptide ions and obtaining mass spectral data;
and wherein in the second mode of operation the method comprises the steps of:
   (i) mass selectively selecting parent peptide ions having a particular mass to charge ratio;
   (ii) fragmenting or reacting at least some or substantially all of the parent peptide ions having a particular mass to charge ratio in a collision, fragmentation or reaction device to form a plurality of fragment, daughter, product or adduct ions; and
   (iii) mass analysing the fragment, daughter, product or adduct ions.

In both cases, in the second mode of operation peptide ions are preferably continually separated according to their ion mobility or rate of change of ion mobility with electric field strength.

The Shotgun fragmentation mode of operation differs from a tandem mass spectrometry or MS/MS mode of operation in that preferably ions are fragmented in an essentially parallel manner i.e. all parent ions which emerge from the ion mobility separator at a particular instance in time are preferably fragmented at essentially the same time. In contrast, in a tandem mass spectrometry or MS/MS mode of operation parent ions are preferably individually selected by a mass filter (e.g. a quadrupole mass filter) and are preferably passed in a serial manner to a collision cell where the parent ions are fragmented. The resulting fragment ions are then preferably mass analysed. The mass filter is then preferably adjusted to selectively transmit a different species of parent ions to the collision cell whereupon the process is preferably repeated.

According to an embodiment the step of enzymatically digesting, chemically reacting or fragmenting a cross-linked protein complex preferably comprises:
enzymatically digesting, chemically reacting or fragmenting the cross-linked protein complex in ¹⁶O-water to form or produce a first plurality of peptides;
enzymatically digesting, chemically reacting or fragmenting the cross-linked protein complex in ¹⁸O-water to form or produce a second plurality of peptides; and
comparing the first plurality of peptides with the second plurality of peptides and selecting as candidate ions for further investigation ions that incorporate more than two ¹⁸O atoms.

According to an embodiment the method may further comprise purifying, separating or fractionating the cross-linked protein complex. The step of purifying, separating or fractionating the cross-linked protein complex preferably further comprises separating components of the cross-linked protein complex by means of chromatography, liquid chromatography or electrophoresis.

There is also disclosed herein a method of mass spectrometry comprising:
digesting, reacting or fragmenting a sample comprising a plurality of parent cross-linked molecules to form or produce a plurality daughter molecules;
ionising at least some of the plurality of daughter molecules to form or produce a plurality of daughter ions, wherein some of the daughter ions comprise cross-linked daughter ions whilst other of the daughter ions comprise non cross-linked daughter ions;
separating at least some of the daughter ions according to their ion mobility or rate of change of ion mobility with electric field strength; and
mass analysing at least some of the daughter ions.

The method preferably further comprises determining, recognising or identifying as candidate daughter ions, daughter ions which have an ion mobility drift time which is substantially faster than the average or mean predicted or experimentally determined drift time for non cross-linked daughter ions.

The method preferably further comprises fragmenting one or more candidate daughter ions to form a plurality of fragment ions and mass analysing the fragment ions.

The method preferably further comprises confirming whether or not one or more of the candidate daughter ions comprise cross-linked ions.

According to another aspect of the present invention there is provided a method of mass spectrometry comprising:
digesting, reacting or fragmenting a sample comprising a plurality of parent molecules to form or produce a plurality of daughter molecules, wherein at least some of the parent molecules comprise two polymer chains covalently linked together by one or more cross-linking bonds;
ionising at least some of the daughter molecules to form or produce a plurality of daughter ions, wherein at least some of the daughter ions comprise cross-linked ions;
separating at least some of the ions according to their ion mobility or rate of change of ion mobility with electric field strength;
mass analysing at least some of the ions;
determining, recognising or identifying as candidate daughter ions, daughter ions which have an ion mobility drift time which is at least 10% faster than the average or mean predicted or experimentally determined drift time for non cross-linked daughter ions;
fragmenting one or more candidate daughter ions to form a plurality of fragment ions and mass analysing the fragment ions; and
identifying the one or more of the candidate daughter ions.

According to the preferred embodiment one or more cross-linked daughter ions are preferably determined, recognised or identified.

There is also disclosed herein a method of mass spectrometry comprising:
digesting, reacting or fragmenting a sample comprising a plurality of conjugated parent molecules to form or produce a plurality daughter molecules, the daughter molecules comprising conjugated and unconjugated daughter molecules;
ionising at least some of the plurality of daughter molecules to form or produce a plurality of daughter ions;
separating at least some of the daughter ions according to their ion mobility or rate of change of ion mobility with electric field strength;
mass analysing at least some of the daughter ions; and
determining or identifying daughter ions which correspond or relate to conjugated daughter molecules at least in part due to the relative drift time or relative ion mobility of the daughter ions.

The parent molecules preferably comprise proteins and/or the daughter molecules preferably comprise peptides.

According to an embodiment of the present invention a method for the determination of protein interaction partners and their site of interaction is preferably provided.

According to the preferred embodiment a method is disclosed which preferably involves cross-linking chemistry followed by enzymatic or chemical cleavage of proteins. This step is preferably followed in turn by analysing resulting peptide ions by ion mobility spectrometry (IMS) coupled with mass spectrometry (MS). The preferred embodiment enables a rapid and sensitive method of protein interaction profiling to be performed.

According to an embodiment of the present invention an analyte protein is cross-linked with an interacting biomolecule which may, for example, comprise a protein, DNA or RNA. The analyte protein is preferably cross-linked to the protein, DNA or RNA using a chemical cross-linking reagent although other embodiments are contemplated wherein the analyte protein may naturally form one or more cross-links with the interacting biomolecule. Cross-linking may also be induced by other means such as exposure to ultra-violet light.

The cross-linked protein complex may optionally be then purified by, for example, chromatographic or electrophoretic based separation methods. According to an embodiment HPLC or 2D-gel electrophoresis may be used to purify the protein complex. The purified protein complex is then preferably subjected to digestion and the resulting peptide digest products are preferably passed to an Electrospray ionisation source for ionisation.

The peptide digest products are preferably ionised in the ion source and the resulting peptide ions are then preferably passed through an ion mobility separator or spectrometer in order to determine their ion mobility in gas phase. The mobility of the peptide ions in gas phase is preferably determined by measuring the transit or drift time of the ions through an ion mobility drift cell. The ion mobility drift cell preferably comprises an ion guide filled with a background gas. Ions are preferably urged through the drift cell and the background gas by, for example, maintaining a DC voltage gradient across the drift cell. According to an embodiment the typical drift time of ions through the drift cell may be of the order of 2-15 ms. However, other embodiments are contemplated wherein the drift time may be < 2 ms or > 15 ms. Once the peptide ions emerge from the drift cell then the peptide ions are preferably passed to a mass analyser in order to be mass analysed. The mass analyser preferably comprises a Time of Flight mass analyser.

The ion mobility data and corresponding mass spectral data are then preferably interrogated in order to look for or search for ions having mass to charge ratios and/or ion mobility drift times which preferably either fall outside of a normal or expected range (which is either observed and/or expected for non cross-linked peptides) or which lie in outlying regions of normal or expected ranges (which are either observed and/or expected for non cross-linked peptides).

The mass spectrometer may according to one embodiment be switched repeatedly between two different modes of operation. In one mode of operation the mass spectrometer is preferably operated in an ion mobility-mass spectrometry ("IMS-MS") mode of operation wherein parent peptide ions are subjected to separation according to their ion mobility in an ion mobility separation stage. The parent peptide ions are then preferably mass analysed.

In the second mode of operation the ions continue to be subjected to ion mobility separation. However, at least some of the ions which emerge from the ion mobility separator are then preferably subjected to fragmentation. The mass spectrometer is preferably operated in a fragmentation mode of operation wherein parent peptide ions are preferably fragmented in a collision cell and the resulting fragment or daughter ions are then preferably mass analysed. The fragmentation of the parent peptide ions and the subsequent mass analysis of the resulting fragment or daughter ions preferably enables further structural information about the parent peptide ions to be elucidated. In the second mode of operation parent peptide ions may be fragmented in parallel (in a manner known as "Shotgun") wherein substantially all peptide ions which emerge from the ion mobility separator are fragmented simultaneously. Alternatively, the parent peptide ions may be fragmented in series (in a tandem mass spectrometry manner or MS/MS) wherein individual species of peptide ions are preferably mass selectively selected by a mass filter and are then individually passed to a collision cell to be fragmented. The resulting fragment ions are preferably mass analysed and the mass filter is then preferably adjusted to mass selectively transmit a different species of parent peptide ions to the collision cell.

Various embodiments of the present invention will now be described, by way of example only, and with reference to the accompanying drawings in which:
Fig. 1 shows a plot of ion mobility drift time versus mass to charge ratio for peptide ions derived from Bovine Serum Albumin ("BSA") wherein the protein was reduced and alkylated to destroy any naturally occurring cross-links between peptides; and
Fig. 2 shows a plot of ion mobility drift time versus mass to charge ratio for peptide ions derived from Bovine Serum Albumin ("BSA") wherein the protein was preserved so that at least some of the resulting peptide ions comprise peptides which are cross-linked by di-sulphide covalent bonds.

According to an embodiment of the present invention two or more different proteins may be mixed to see whether or not they will cross-link. For example, a known protein may be mixed with a complex mixture of other proteins which are derived, for example, from a cell line. The intention is to see which proteins form cross-links with the known protein. The protein-protein interactions are performed *in vitro.*

According to a less preferred embodiment an artificially produced biomolecule may be compared with a naturally existing biomolecule. For example, it may be desired to see how an artificially produced biomolecule (e.g. a drug or biochemical agent) interacts with the naturally existing biomolecule (e.g. a protein). Comparison of how proteins interact in and out of a complex mixture enables the strength of a protein-protein or protein-biomolecular interaction to be determined and helps to indicate whether or not an interaction may be biologically relevant.

According to the preferred embodiment one or more samples may be subjected to enzymatic or chemical treatment in order to produce a plurality of peptides. At least some of the resulting peptides are preferably cross-linked.

The digested sample which preferably comprises a plurality of different peptides is preferably ionised by an Electrospray ion source. The resulting peptide ions are then preferably passed to an ion mobility separator or spectrometer. The peptide ions are then preferably temporally separated based upon their ion mobility in the gas phase. The drift time through the ion mobility separator or spectrometer is preferably recorded in one of a number of discrete drift time time bins. According to one embodiment the mobility of ions through the ion mobility separator may be recorded in one of 200 discrete time bins.

Ions falling within each discrete drift time bin are preferably mass analysed by a Time of Flight mass spectrometer to determine the mass to charge ratio and charge of each molecular species.

According to an embodiment the ions falling within each time bin may also be analysed by tandem mass spectrometry ("MS/MS") or by parallel Collision Induced Dissociation ("CID") (high/low collision energy) to obtain structural information of each ion species.

Identification or recognition of chemically cross-linked peptides may be performed by analysing the difference in the ion mobility of certain peptide ions relative to other peptides/ions which are not cross-linked. For example, ion mobility separations have been investigated to determine cross-linked peptides in a sample of Bovine Serum Albumin ("BSA"). The investigations have shown that some of the largest di-sulphide cross-linked peptide ions can be detected or recognised on the basis that they have a substantially faster or quicker ion mobility drift time relative to other ions having substantially similar mass to charge ratios. This indicates that these large peptides with molecular weights of the order of 5000 Da are folded with the result that their cross-sectional area is reduced.

The acquired ion mobility drift time data and corresponding mass, mass to charge ratio, intensity and retention time (or any combination of these) information may be displayed in the form of a graphical plot.

Comparison of data (e.g. either IMS data or IMS-MS data) from a sample that may contain cross-linked peptides with a sample which was digested without any chemically cross-linked peptides can be used in a subtractive manner to determine differences in ion mobility and/or mass to charge ratios between the two samples.

Further experiments may be performed to obtain MS/MS or other structural information of those components or ions that exhibit differences in either their ion mobility and/or their mass or mass to charge ratio between samples in order to elucidate the identity of any observed cross-linked peptide components.

According to an embodiment the approach according to the preferred embodiment can be used to discover cross-linked peptides and hence proteins which form cross-links in order to understand which proteins interact. The preferred embodiment also enables researchers to investigate and to gain understanding as to how protein interactions may be indicative of a particular disease or the progression of a particular disease. The preferred method also enables the specific sites (e.g. amino acids) that interact between protein species to be identified.

In order to illustrate various aspects of the present invention two different samples of Bovine Serum Albumin ("BSA") were prepared and investigated.

Bovine Serum Albumin is a protein that folds naturally due to the presence of several cysteine amino acids in its sequence. The cysteine amino acids contain a -SH terminal group and pairs of these will link naturally to form di-sulphide bridges. This results in the protein becoming folded to take up a particular reproducible shape. This process of self-bonding is slightly different to the process of two different proteins reacting within a reagent and linking to each other via one or more bridges. Nonetheless, the experimental results discussed below help to illustrate various aspects of the preferred embodiment.

When two different protein molecules react and bind together they can do so by linking at cysteine amino acids to form covalent di-sulphide bridges in a similar manner to the self-bonding process which occurs with Bovine Serum Albumin. However, two different proteins can also link at other amino acid sites. The alternative bonding sites are not so easily predictable.

A first sample of Bovine Serum Albumin was prepared with the intention of destroying any naturally occurring di-sulphide bonds. The first sample was prepared by solubilising the sample of Bovine Serum Albumin in 50 mM of Ammonium Bicarbonate. The sample was then reduced with 10 mM of dithiothreitol ("DTT") reducing agent. The sample was then heated at 60°C for 30 minutes and was allowed to cool. The condensate was then spun down. The sample was then alkylated with iodoacetamide ("IAA") to a final concentration of 10 mM. The sample was then left in the dark for 30 minutes at room temperature. The sample was then digested with trypsin which was added at a ratio of 50:1 by concentration (protein:trypsin). The first sample was then allowed to incubate overnight at 37°C.

A second sample of Bovine Serum Albumin was prepared with the intention of preserving any naturally occurring di-sulphide bonds. In order to preserve any di-sulphide bonds, the second sample was firstly solubilised in 50 mM of ammonium bicarbonate. The second sample was then digested with trypsin which was added at a ratio of 50:1 by concentration (protein:trypsin). The second sample was then allowed to incubate overnight at 37°C.

Accordingly, whereas the first sample was reduced with 10 mM of dithiothreitol ("DTT") reducing agent and was then alkylated with 10 mM iodoacetamide, the second sample was not subjected to either reduction or alkylation. Any naturally occurring di-sulphide bonds between peptides in the second sample were therefore preserved.

The first and second samples were then analysed in a manner according to the preferred embodiment using a SYNAPT (RTM) HDMS (RTM) mass spectrometer available from WATERS (RTM), Milford MA (USA). The first and second samples were analysed by introducing the samples, in turn, into the Electrospray ion source of the mass spectrometer. The resulting parent ions were then guided through various ion guiding stages of the mass spectrometer to an ion mobility separation stage. The drift time of peptide ions through the ion mobility separation stage of the mass spectrometer was then recorded. Peptide ions which emerged from the ion mobility separation stage of the mass spectrometer were then passed to an orthogonal acceleration Time of Flight mass analyser which was arranged to mass analyse the peptide ions.

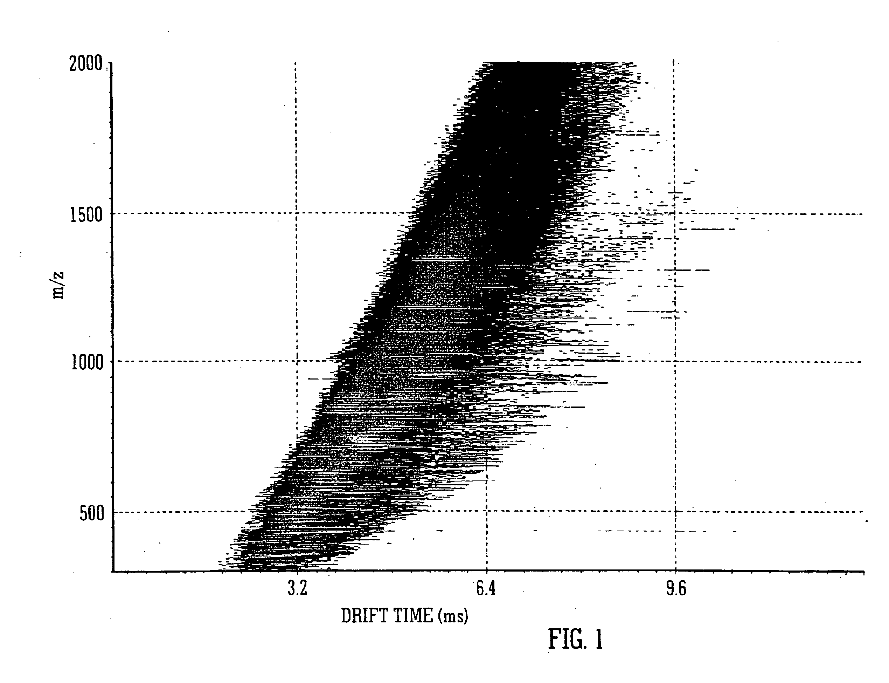

Fig. 1 shows a plot of the drift time of peptide ions derived from the first sample through the ion mobility separation stage of the mass spectrometer as a function of mass to charge ratio. It can be seen that the ion mobility data separates out into a number of distinct bands or regions. Each band or region corresponds to ions having a particular charge state.

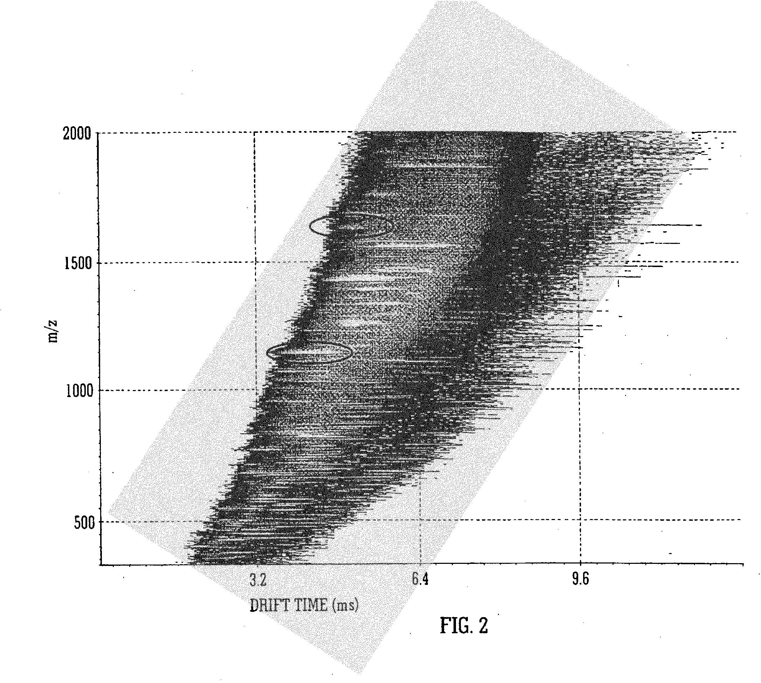

Fig. 2 shows a plot of the drift time of peptide ions derived from the second sample through the ion mobility separation stage of the mass spectrometer as a function of mass to charge ratio. It can be seen that the data again separates out into a number of distinct bands or regions. Each band or region corresponds to ions having a particular charge state.

A couple of outlying regions are circled in Fig. 2. The circled regions relate to ions which have substantially shorter or faster drift times through the ion mobility separation stage of the mass spectrometer compared with other peptide ions which have a comparable mass to charge ratio. Subsequent investigation of these ions suggests that these ions relate to peptides which were cross-linked by di-sulphide bonds.

Fig. 2 illustrates an important aspect of the preferred embodiment, namely that cross-linked peptide ions and other cross-linked biomolecular ions can be identified according to the method of the preferred embodiment by recognising, identifying or searching for ions which have a drift time through the ion mobility separation stage of a mass spectrometer which is substantially shorter or faster than the average or median drift time predicted for non cross-linked peptide ions having substantially the same or comparable mass to charge ratios and/or charge state.

Although the present invention has been described with reference to preferred embodiments, it will be understood by those skilled in the art that various changes in form and detail may be made to the particular embodiments discussed above without departing from the scope of the invention as set forth in the accompanying claims.

## Claims

1. A method of mass spectrometry comprising:
enzymatically digesting, chemically reacting or fragmenting a covalently cross-linked protein complex to form or produce a plurality of peptides, wherein said peptides comprise a mixture of cross-linked peptides and non cross-linked peptides;
ionising at least some of said plurality of peptides to form a plurality of peptide ions;
separating at least some of said peptide ions according to their ion mobility or their rate of change of ion mobility with electric field strength and obtaining ion mobility data; and
mass analysing at least some of said peptide ions and obtaining mass spectral data.

2. A method as claimed in claim 1, further comprising:
distinguishing between cross-linked peptides or cross-linked peptide ions and non cross-linked peptides or non cross-linked peptide ions; and
further comprising:
(i) determining, recognising or identifying one or more cross-linked peptides or cross-linked peptide ions; and/or
(ii) determining, recognising or identifying one or more non cross-linked peptides or non cross-linked peptide ions;
wherein said step of determining, recognising or identifying said one or more cross-linked peptides or cross-linked peptide ions comprises analysing said ion mobility data and/or said mass spectral data; and
wherein said step of determining, recognising or identifying said one or more non cross-linked peptides or non cross-linked peptide ions comprises analysing said ion mobility data and/or said mass spectral data.

3. A method as claimed in claim 1 or 2, further comprising analysing said ion mobility data and/or said mass spectral data to determine, recognise or identify peptides or peptide ions which have:
(i) an ion mobility characteristic and/or a mass spectral characteristic which falls outside of a standard, normal or predicted range; and/or
(ii) an ion mobility characteristic and/or a mass spectral characteristic which falls towards an upper limit or towards a lower limit of a standard, normal or predicted range; and/or
(iii) an outlying ion mobility characteristic and/or an outlying mass spectral characteristic; and/or
(iv) an ion mobility drift time versus mass to charge ratio relationship or dependence which falls between or outside one or more ranges or bands predicted for non cross-linked peptides; and/or
(v) an ion mobility drift time versus mass to charge ratio relationship or dependence which falls within one or more ranges or bands predicted for cross-linked peptides.

4. A method as claimed in any preceding claim, further comprising analysing said ion mobility data and/or said mass spectral data:
(i) to determine, recognise or identify peptides or peptide ions which have an ion mobility drift time which is x% faster or slower than the average or mean predicted drift time for non cross-linked ions or other ions having a comparable mass to charge ratio and/or charge state; and/or
(ii) to determine, recognise or identify peptides or peptide ions which have a mass or mass to charge ratio which is x% higher or lower than the average or mean predicted or experimentally determined mass or mass to charge ratio for non cross-linked ions or other ions having a comparable drift time or ion mobility;
wherein x is selected from the group consisting of: (i) < 5; (ii) 5-10; (iii) 10-15; (iv) 15-20; (v) 20-25; (vi) 25-30; (vii) 30-35; (viii) 35-40; (ix) 40-45; (x) 45-50; (xi) 50-55; (xii) 55-60; (xiii) 60-65; (xiv) 65-70; (xv) 70-75; (xvi) 75-80; (xvii) 80-85; (xviii) 85-90; (xix) 90-95; (xx) 95-100; (xxi) 100-110; (xxii) 110-120; (xxiii) 120-130; (xxiv) 130-140; (xxv) 140-150; (xxvi) 150-160; (xxvii) 160-170; (xxviii) 170-180; (xxix) 180-190; (xxx) 190-200; and (xxxi) > 200.

5. A method as claimed in any preceding claim, wherein said method further comprises the step of forming said cross-linked protein complex prior to said step of enzymatically digesting, chemically reacting or fragmenting said cross-linked protein complex.

6. A method as claimed in any preceding claim, further comprising repeatedly switching between a first mode of operation and a second mode of operation, wherein:
in said first mode of operation said method comprises the steps of:
(i) separating at least some of said plurality of peptide ions according to their ion mobility or rate of change of ion mobility with electric field strength and obtaining ion mobility data; and
(ii) mass analysing at least some of said peptide ions and obtaining mass spectral data;
and wherein in said second mode of operation said method comprises the steps of:
(i) fragmenting or reacting at least some or substantially all of said plurality of peptide ions in a collision, fragmentation or reaction device to form a plurality of fragment, daughter, product or adduct ions; and
(ii) mass analysing said fragment, daughter, product or adduct ions.

7. A method as claimed in any preceding claim, further comprising repeatedly switching between a first mode of operation and a second mode of operation, wherein:
in said first mode of operation said method comprises the steps of:
(i) separating at least some of said plurality of peptide ions according to their ion mobility or rate of change of ion mobility with electric field strength and obtaining ion mobility data; and
(ii) mass analysing at least some of said peptide ions and obtaining mass spectral data;
and wherein in said second mode of operation said method comprises the steps of:
(i) mass selectively selecting parent peptide ions having a particular mass to charge ratio;
(ii) fragmenting or reacting at least some or substantially all of said parent peptide ions having a particular mass to charge ratio in a collision, fragmentation or reaction device to form a plurality of fragment, daughter, product or adduct ions; and
(iii) mass analysing said fragment, daughter, product or adduct ions.

8. A method of mass spectrometry comprising:
digesting, reacting or fragmenting a sample comprising a plurality of parent molecules to form or produce a plurality of daughter molecules, wherein at least some of said parent molecules comprise two polymer chains covalently linked together by one or more cross-linking bonds;
ionising at least some of said daughter molecules to form or produce a plurality of daughter ions, wherein at least some of said daughter ions comprise cross-linked ions;
separating at least some of said ions according to their ion mobility or rate of change of ion mobility with electric field strength;
mass analysing at least some of said ions;
determining, recognising or identifying as candidate daughter ions, daughter ions which have an ion mobility drift time which is at least 10% faster than the average or mean predicted or experimentally determined drift time for non cross-linked daughter ions;
fragmenting one or more candidate daughter ions to form a plurality of fragment ions and mass analysing said fragment ions; and
identifying said one or more of said candidate daughter ions.

9. A method as claimed in claim 8, wherein said parent molecules comprise proteins and/or said daughter molecules comprise peptides.

## Patentansprüche

1. Verfahren der Massenspektrometrie, umfassend:
enzymatisches Verdauen, chemisches Reagieren oder Fragmentierung eines covalent quervernetzten Proteinkomplexes zur Ausbildung oder Erzeugung mehrerer Peptide, wobei die Peptide eine Mischung von quervernetzten Peptiden und nicht-quervernetzten Peptiden umfassen;
Ionisieren mindestens einiger der mehreren Peptide zur Ausbildung mehrerer Peptidionen;
Trennen mindestens einiger der Peptidionen entsprechend ihrer Ionenmobilität oder ihrer Ionenmobilitätänderungsrate mit elektrischer Feldstärke und Erhalten von Ionenmobilitätsdaten und
Massenanalysieren mindestens einiger der Peptidionen und Erhalten von Massenspektraldaten.

2. Verfahren nach Anspruch 1, ferner umfassend:
Unterscheiden zwischen quervernetzten Peptiden oder quervernetzten Peptidionen und nicht-quervernetzten Peptiden oder nicht-quervernetzten Peptidionen und
ferner umfassend:
(i) Bestimmen, Erkennen oder Identifizieren einer oder mehrerer quervernetzter Peptide oder quervernetzter Peptidionen und/oder
(ii) Bestimmen, Erkennen oder Identifizieren einer oder mehrerer nicht-quervernetzter Peptide oder nichtquervernetzter Peptidionen;
wobei der Schritt des Bestimmens, Erkennens oder Identifizierens des einen oder der mehreren quervernetzten Peptide oder quervernetzten Peptidionen Analysieren der Ionenmobilitätsdaten und/oder der Massenspektraldaten umfasst und
wobei der Schritt des Bestimmens, Erkennens oder Identifizieren des einen oder der mehreren nicht-quervernetzten Peptide oder nicht-quervernetzten Peptidionen Analysieren der Ionenmobilitätsdaten und/oder der Massenspektraldaten umfasst.

3. Verfahren nach Anspruch 1 oder 2, ferner umfassend Analysieren der Ionenmobilitätsdaten und/oder Massenspektraldaten zum Bestimmen, Erkennen oder Identifizieren von Peptiden oder Peptidionen, die:
(i) eine Ionenmobilitätscharakteristik und/oder eine Massenspektralcharakteristik, die außerhalb eines standardmäßigen, normalen oder vorhergesagten Bereichs fällt; und/oder
(ii) eine Ionenmobilitätscharakteristik und/oder eine Massenspektralcharakteristik, die in Richtung einer oberen Grenze oder in Richtung einer unteren Grenze eines standardmäßigen, normalen oder vorhergesagten Bereichs fällt; und/oder
(iii) eine Ausreißerionenmobilitätscharakteristik und/oder eine Ausreißermassenspektralcharakteristik und/oder
(iv) eine Beziehung oder Abhängigkeit der Ionenmobilität-Driftzeit gegenüber dem Masse-zu-Ladung-Verhältnis, die innerhalb oder außerhalb eines oder mehrerer Bereiche oder Bände fällt, die für nicht-quervernetzte Peptide vorhergesagt werden; und/oder
(v) eine Beziehung oder Abhängigkeit der Ionenmobilität-Driftzeit gegenüber dem Masse-zu-Ladung-Verhältnis, die innerhalb eines oder mehrerer Bereiche oder Bände fällt, die für quervernetzte Peptide vorhergesagt werden,
aufweisen.

4. Verfahren nach einem vorhergehenden Anspruch, ferner umfassend Analysieren der Ionenmobilitätsdaten und/oder Massenspektraldaten:
(i) zum Bestimmen, Erkennen oder Identifizieren von Peptiden oder Peptidionen, die eine Ionenmobilität-Driftzeit aufweisen, die x% schneller oder langsamer als die durchschnittliche oder mittlere vorhergesagte Driftzeit für nicht-quervernetzte Ionen oder andere Ionen mit einem vergleichbaren Masse-zu-Ladung-Verhältnis und/oder Ladungszustand ist; und/oder
(ii) zum Bestimmen, Erkennen oder Identifizieren von Peptiden oder Peptidionen, die eine Masse oder ein Masse-zu-Ladung-Verhältnis aufweisen, die bzw. das x% höher oder niedriger als die durchschnittliche oder mittlere vorhergesagte oder experimentell bestimmte Masse oder das durchschnittliche oder mittlere vorhergesagte oder experimentell bestimmte Masse-zu-Ladung-Verhältnis für nicht-quervernetzte Ionen oder andere Ionen mit einer vergleichbaren Driftzeit oder Ionenmobilität ist;
wobei x ausgewählt ist aus der Gruppe bestehend aus: (i) <5; (ii) 5-10; (iii) 10-15; (iv) 15-20; (v) 20-25; (vi) 25-30; (vii) 30-35; (viii) 35-40; (ix) 40-45; (x) 45-50; (xi) 50-55; (xii) 55-60; (xiii) 60-65; (xiv) 65-70; (xv) 70-75; (xvi) 75-80; (xvii) 80-85; (xviii) 85-90; (xix) 90-95; (xx) 95-100; (xxi) 100-110; (xxii) 110-120; (xxiii) 120-130; (xxiv) 130-140; (xxv) 140-150; (xxvi) 150-160; (xxvii) 160-170; (xxviii) 170-180; (xxix) 180-190; (xxx) 190-200; und (xxxi) >200.

5. Verfahren nach einem vorhergehenden Anspruch, wobei das Verfahren ferner den Schritt Ausbilden des quervernetzten Proteinkomplexes vor dem Schritt enzymatisches Verdauen, chemisches Reagieren oder Fragmentieren des quervernetzten Proteinkomplexes umfasst.

6. Verfahren nach einem vorhergehenden Anspruch, ferner umfassend wiederholtes Umschalten zwischen einem ersten Betriebsmodus und einem zweiten Betriebsmodus, wobei:
im ersten Betriebsmodus das Verfahren die folgenden Schritte umfasst:
(i) Trennen mindestens einiger der mehreren Peptidionen gemäß ihrer Ionenmobilität oder ihrer Ionenmobilitätsänderungsrate mit elektrischer Feldstärke und Erhalten von Ionenmobilitätsdaten und
(ii) Massenanalysieren mindestens einiger der Peptidionen und Erhalten von Massespektraldaten;
und wobei im zweiten Betriebsmodus das Verfahren die folgenden Schritte umfasst:
(i) Fragmentieren oder Reagieren mindestens einiger oder im Wesentlichen aller der mehreren Peptidionen in einer Kollisions-, Fragmentierungs- oder Reaktionseinrichtung zur Ausbildung mehrerer Fragment-, Tochter-, Produkt- oder Adduktionen und
(ii) Massenanalysieren der Fragment-, Tochter-, Produkt- oder Adduktionen.

7. Verfahren nach einem vorhergehenden Anspruch, ferner umfassend wiederholtes Umschalten zwischen einem ersten Betriebsmodus und einem zweiten Betriebsmodus, wobei:
im ersten Betriebsmodus das Verfahren die folgenden Schritte umfasst:
(i) Trennen mindestens einiger der mehreren Peptidionen gemäß ihrer Ionenmobilität oder ihrer Ionenmobilitätsänderungsrate mit elektrischer Feldstärke und Erhalten von Ionenmobilitätsdaten und
(ii) Massenanalysieren mindestens einiger der Peptidionen und Erhalten von Massespektraldaten;
und wobei im zweiten Betriebsmodus das Verfahren die folgenden Schritte umfasst:
(i) massenselektives Auswählen von Elternpeptidionen mit einem bestimmten Masse-zu-Ladung-Verhältnis;
(ii) Fragmentieren oder Reagieren mindestens einiger oder im Wesentlichen aller Elternpeptidionen mit einem bestimmten Masse-zu-Ladung-Verhältnis in einer Kollisions-, Fragmentierungs- oder Reaktionseinrichtung zur Ausbildung mehrerer Fragment-, Tochter-, Produkt- oder Adduktionen und
(iii) Massenanalysieren der Fragment-, Tochter-, Produkt- oder Adduktionen.

8. Verfahren der Massenspektrometrie, umfassend:
Verdauen, Reagieren oder Fragmentieren einer Probe, die mehrere Elternmoleküle umfasst, zur Ausbildung oder Erzeugung mehrerer Tochtermoleküle, wobei mindestens einige der Elternmoleküle zwei Polymerketten umfassen, die mit einer oder mehreren Quervernetzungen kovalent zusammengebunden sind;
Ionisieren mindestens einiger der Tochtermoleküle zur Ausbildung oder Erzeugung mehrerer Tochterionen, wobei mindestens einige der Tochterionen quervernetzte Ionen umfassen;
Trennen mindestens einiger der Ionen entsprechend ihrer Ionenmobilität oder ihrer Ionenmobilitätsänderungsrate mit elektrischer Feldstärke;
Massenanalysieren mindestens einiger der Ionen;
Bestimmen, Erkennen oder Identifizieren von Tochterionen, die eine Ionenmobilität-Driftzeit aufweisen, die mindestens 10% schneller als die durchschnittliche oder mittlere vorhergesagte oder experimentell bestimmte Driftzeit für nicht-quervernetzte Tochterionen ist, als mögliche Tochterionen;
Fragmentieren einer oder mehrerer möglicher Tochterionen zur Ausbildung mehrerer Fragmentionen und Massenanalysieren der Fragmentionen und
Identifizieren der einen oder mehreren möglichen Tochterionen.

9. Verfahren nach Anspruch 8, wobei die Elternmoleküle Proteine umfassen und/oder die Tochtermoleküle Peptide umfassen.

## Revendications

1. Procédé de spectrométrie de masse comprenant :
la digestion enzymatique, la réaction chimique ou la fragmentation d'un complexe protéique réticulé de façon covalente pour former ou produire une pluralité de peptides, lesdits peptides comprenant un mélange de peptides réticulés et de peptides non réticulés ;
l'ionisation d'au moins certains de ladite pluralité de peptides pour former une pluralité d'ions peptidiques ;
la séparation d'au moins certains desdits ions peptidiques en fonction de leur mobilité ionique ou leur vitesse de variation de mobilité ionique avec l'intensité du champ électrique et l'obtention de données de mobilité ionique ; et
l'analyse de masse d'au moins certains desdits ions peptidiques et l'obtention de données spectrales de masse.

2. Procédé selon la revendication 1, comprenant en outre :
la distinction entre les peptides réticulés ou les ions peptidiques réticulés et les peptides non réticulés ou les ions peptidiques non réticulés ; et
comprenant en outre :
(i) la détermination, la reconnaissance ou l'identification d'un ou plusieurs peptides réticulés ou ions peptidiques réticulés ; et/ou
(ii) la détermination, la reconnaissance ou l'identification d'un ou plusieurs peptides non réticulés ou ions peptidiques non réticulés ;
dans lequel ladite étape de détermination, reconnaissance ou identification desdits un ou plusieurs peptides réticulés ou ions peptidiques réticulés comprend l'analyse desdites données de mobilité ionique et/ou desdites données spectrales de masse ; et
dans lequel ladite étape de détermination, reconnaissance ou identification desdits un ou plusieurs peptides non réticulés ou ions peptidiques non réticulés comprend l'analyse desdites données de mobilité ionique et/ou desdites données spectrales de masse.

3. Procédé selon la revendication 1 ou 2, comprenant en outre l'analyse desdites données de mobilité ionique et/ou desdites données spectrales de masse pour déterminer, reconnaître ou identifier des peptides ou ions peptidiques qui ont :
(i) une caractéristique de mobilité ionique et/ou une caractéristique spectrale de masse qui se situent à l'extérieur d'une gamme standard, normale ou prédite ; et/ou
(ii) une caractéristique de mobilité ionique et/ou une caractéristique spectrale de masse qui se situent vers une limite supérieure ou vers une limite inférieure d'une gamme standard, normale ou prédite ; et/ou
(iii) une caractéristique de mobilité ionique périphérique et/ou une caractéristique spectrale de masse périphérique ; et/ou
(iv) une relation ou dépendance du temps de dérive de mobilité ionique envers le rapport masse sur charge qui se situe entre ou à l'extérieur d'une ou plusieurs gammes ou bandes prédites pour des peptides non réticulés ; et/ou
(v) une relation ou dépendance du temps de dérive de mobilité ionique envers le rapport masse sur charge qui se situe à l'intérieur d'une ou plusieurs gammes ou bandes prédites pour des peptides réticulés.

4. Procédé selon l'une quelconque des revendications précédentes, comprenant en outre l'analyse desdites données de mobilité ionique et/ou desdites données spectrales de masse :
(i) pour déterminer, reconnaître ou identifier des peptides ou ions peptidiques qui ont un temps de dérive de mobilité ionique qui est x % plus rapide ou plus lent que le temps de dérive moyen ou moyen prédit pour des ions non réticulés ou d'autres ions ayant un rapport masse sur charge et/ou un état de charge comparables ; et/ou
(ii) pour déterminer, reconnaître ou identifier des peptides ou ions peptidiques qui ont une masse ou un rapport masse sur charge qui est x % plus grand ou plus petit que la masse ou le rapport masse sur charge moyen ou moyen prédit ou déterminé expérimentalement pour des ions non réticulés ou d'autres ions ayant un temps de dérive ou une mobilité ionique comparable ;
dans lequel x est choisi dans le groupe constitué par : (i) < 5 ; (ii) 5-10 ; (iii) 10-15 ; (iv) 15-20 ; (v) 20-25 ; (vi) 25-30 ; (vii) 30-35 ; (viii) 35-40 ; (ix) 40-45 ; (x) 45-50 ; (xi) 50-55 ; (xii) 55-60 ; (xiii) 60-65 ; (xiv) 65-70 ; (xv) 70-75 ; (xvi) 75-80 ; (xvii) 80-85 ; (xviii) 85-90 ; (xix) 90-95 ; (xx) 95-100 ; (xxi) 100-110 ; (xxii) 110-120 ; (xxiii) 120-130 ; (xxiv) 130-140 ; (xxv) 140-150 ; (xxvi) 150-160 ; (xxvii) 160-170 ; (xxviii) 170-180 ; (xxix) 180-190 ; (xxx) 190-200 ; et (xxxi) > 200.

5. Procédé selon l'une quelconque des revendications précédentes, ledit procédé comprenant en outre l'étape de formation dudit complexe protéique réticulé avant ladite étape de digestion enzymatique, réaction chimique ou fragmentation dudit complexe protéique réticulé.

6. Procédé selon l'une quelconque des revendications précédentes, comprenant en outre le basculement répété entre un premier mode de fonctionnement et un deuxième mode de fonctionnement, dans lequel :
dans ledit premier mode de fonctionnement ledit procédé comprend les étapes consistant à :
(i) séparer au moins certains de ladite pluralité d'ions peptidiques en fonction de leur mobilité ionique ou vitesse de variation de mobilité ionique avec l'intensité du champ électrique et obtenir des données de mobilité ionique ; et
(ii) analyser la masse d'au moins certains desdits ions peptidiques et obtenir des données spectrales de masse ;
et dans lequel dans ledit deuxième mode de fonctionnement ledit procédé comprend les étapes consistant à :
(i) fragmenter ou faire réagir au moins certains ou pratiquement la totalité de ladite pluralité d'ions peptidiques dans un dispositif de collision, fragmentation ou réaction pour former une pluralité d'ions fragments, fils, produits ou adduits ; et
(ii) analyser la masse desdits ions fragments, fils, produits ou adduits.

7. Procédé selon l'une quelconque des revendications précédentes, comprenant en outre le basculement répété entre un premier mode de fonctionnement et un deuxième mode de fonctionnement, dans lequel :
dans ledit premier mode de fonctionnement ledit procédé comprend les étapes consistant à :
(i) séparer au moins certains de ladite pluralité d'ions peptidiques en fonction de leur mobilité ionique ou vitesse de variation de mobilité ionique avec l'intensité du champ électrique et obtenir des données de mobilité ionique ; et
(ii) analyser la masse d'au moins certains desdits ions peptidiques et obtenir des données spectrales de masse ;
et dans lequel dans ledit deuxième mode de fonctionnement ledit procédé comprend les étapes consistant à :
(i) choisir sélectivement en fonction de la masse des ions peptidiques parents ayant un rapport masse sur charge particulier ;
(ii) fragmenter ou faire réagir au moins certains ou pratiquement la totalité desdits ions peptidiques parents ayant un rapport masse sur charge particulier dans un dispositif de collision, fragmentation ou réaction pour former une pluralité d'ions fragments, fils, produits ou adduits ; et
(iii) analyser la masse desdits ions fragments, fils, produits ou adduits.

8. Procédé de spectrométrie de masse comprenant :
la digestion, la réaction ou la fragmentation d'un échantillon comprenant une pluralité de molécules parentes pour former ou produire une pluralité de molécules filles, au moins certaines desdites molécules parentes comprenant deux chaînes polymères liées de façon covalente par une ou plusieurs liaisons de réticulation ;
l'ionisation d'au moins certaines desdites molécules filles pour former ou produire une pluralité d'ions fils, au moins certains desdits ions fils comprenant des ions réticulés ;
la séparation d'au moins certains desdits ions en fonction de leur mobilité ionique ou vitesse de variation de mobilité ionique avec l'intensité du champ électrique ;
l'analyse de masse d'au moins certains desdits ions ;
la détermination, la reconnaissance ou l'identification, comme ions fils candidats, d'ions fils qui ont un temps de dérive de mobilité ionique qui est au moins 10 % plus rapide que le temps de dérive moyen ou moyen prédit ou déterminé expérimentalement pour des ions fils non réticulés ;
la fragmentation d'un ou plusieurs ions fils candidats pour former une pluralité d'ions fragments et l'analyse de masse desdits ions fragments ; et
l'identification desdits un ou plusieurs desdits ions fils candidats.

9. Procédé selon la revendication 8, dans lequel lesdites molécules parentes comprennent des protéines et/ou lesdites molécules filles comprennent des peptides.
